# EUROPEAN PATENT APPLICATION

(11) **EP 4 613 838 A1**
(43) Date of publication of application: **10.09.2025**
(21) Application number: 24162392.5
(22) Date of filing: 08.03.2024
(51) Int. Cl.: C12M 1/00, C12M 3/00, C12M 1/36

(54) **AUTOMATED SETTLE PLATE HANDLER**

(71) Applicant: MBV AG, 8712 Staefa (CH)
(72) Inventor: ARMBRECHT, Lucas, 8706 Meilen (CH); KELLER, Daniel, 8640 Rapperswil (CH)
(74) Representative: Schulz Junghans Patentanwälte PartGmbB

(57) **Abstract**

The present invention relates to a plate handling device (1) for handling plates (2), the respective plate (2) accommodating a nutrient medium (3) and being covered by a lid (4). Furthermore, the invention relates to a method for automatically handling such plates (2).

## Description

The present invention relates to an automatic plate handling device and to a corresponding method.

Particularly, the present invention is in the field of particle collection and sampling as well as analysis of particles of all kinds. Especially, it is related to instruments for characterization of gas or air quality as necessary in food and beverage industries or clean or controlled environments such as cleanrooms and manufacturing environments. Examples for such clean environments are production lines in the pharmaceutical industries, where air quality must be constantly observed and tested.

Microbial air sampling thereby refers to the collection of microbe-carrying particles on a growth medium (e.g. agar plate) for subsequent detection. In most cases detection is conducted after incubation and growth of the collected microorganisms into visible colonies.

There generally exist regulatory limits on how many and which organisms are tolerable in a specific environment and in case limits are exceeded, production lots of pharmaceuticals, as one example, may have to be discarded. Hence analysis of the air quality with a focus on microbial contamination is a critical quality indicator for product release in multiple large industries. All instruments in this field must quantify microbial contamination with highest precision and are standardized in ISO 14698-1, 14698-2 and EN 17141.

Passive microbial air sampling is the simplest and most widespread form of air monitoring techniques and relies on gravitational sedimentation of microbe-carrying particles onto an exposed nutrient plate surface. Thereby, an agar plate covered by a lid is placed onto a surface in the controlled environment, the lid is manually removed and the agar is exposed to the air. Herein, dry-out of the exposed plate usually limits the maximum exposure time of a single plate to at most 4h. As a result, frequent manual plate changes have to be carried out, which add to the overall process time, high personnel costs and additionally poses a contamination risk in itself.

Particularly, active microbial air sampling instead uses a directed air flow to impact airborne particles onto an exposed nutrient plate surface. Here, a defined air volume is sampled onto each plate with a set flow rate. While the output of passive microbial air sampling yields a result in viable microbes collected per time per area, active microbial air sampling yields a result in viable microbes per air volume. As a result, both forms of microbial air sampling are frequently complementing each other for air quality monitoring purposes.

So far, manual placement and collection of plates in the above-stated environment/devices is known in the prior art. Typically devices used for automated microbial air sampling involve only a single agar plate. Particularly, US20220380710A1 discloses the use of a robotic arm in a fully automated production line to perform the plate handling. Furthermore, nutrient plate or tissue handling solutions for other purposes are known and described in US20130183132A2, DE102009016364, and US20030044991A1.

Typically, known devices and processes require a manual workflow or frequent manual intervention and interruption of the manufacturing process during that time. Further, there is a lack of traceability, particularly limited to manual notes rather than automatic logging of device actions. Due to manual operation the risk of contamination and error occurrence increases correspondingly. Further, only a single plate is exposed on demand. Using multiple devices one after the other however leads to different testing positions and a large foot-print.

Therefore, based on the above, the problem to be solved by the present invention is to provide a plate handling device for handling plates and a corresponding method for handling plates that mitigates the above-mentioned difficulties.

This problem is solved by a plate handling device for handling plates according to claim 1 and by a method according to claim 14.

Preferred embodiments of these aspects of the present invention are stated in the corresponding dependent claims and are also described below. A further aspect of the present invention relates to a microbial air sampling device having the features of claim 13.

According to claim 1, a plate handling device for handling plates is disclosed, the respective plate preferably accommodating a nutrient medium and being covered by a lid, wherein the plate handling device comprises:
- a first platform being moveable (particularly up and down in a vertical direction) and configured to carry a plurality of said plates stacked on top of one another,
- a second platform being moveable (particularly up and down in a vertical direction),
- a gripper being moveable (e.g. back and forth in a horizontal direction between the first and the second platform), wherein the plate handling device is configured to grip (particularly by means of the gripper) an (e.g. topmost) plate carried by the first platform, move this plate (e.g. along the horizontal direction) to the second platform and to arrange it thereon, and wherein the plate handling device is further configured to remove the lid of this plate to expose the plate, particularly its nutrient medium, to the ambient atmosphere for a pre-defined second time.

In the framework of the present invention, the feature according to which the first and second platform are movable up and down does not necessarily require that the movements of the platforms are purely vertical. The movement of the respective platform can also comprise components in directions that are not vertical. Particularly, an up or down movement may also be generated by a rotary or spiral movement of the first or second platform. Thus, particularly, up and down movements with respect to the platforms include all movements of the respective platform that change a position of the respective platform with respect to the vertical axis. Similarly, the fact that the gripper can move in the horizontal direction does not necessarily mean that the movement of the gripper is purely horizontally, but may comprise further components/directions. However, according to an embodiment, the movement of the gripper can be linear in the horizontal direction (e.g. back and forth).

Particularly, in an embodiment, the plate handling device is configured to let the gripper grip the (e.g. topmost) plate carried by the first platform. Further, in an embodiment, the plate handling device is further configured to remove the lid of this plate with help of the gripper. In yet a further embodiment of the plate handling device, the gripper is movable back and forth in a horizontal direction.

Furthermore, in an embodiment, the plate handling device can be configured to remove the lid of this plate when this plate is arranged on the first platform (i.e. prior to moving the plate to the second platform) or on the second platform (i.e. after moving the plate from the first platform to the second platform).

Thus, advantageously, the present invention allows automation of the otherwise manual plate exchange process for passive environmental monitoring. Particularly, manual interventions are reduced and process stability and data integrity can be facilitated. Furthermore, particularly, the proposed invention requires a comparatively small footprint and offers a high flexibility compared to alternative approaches.

According to an embodiment of the plate handling device, for removing the lid, the plate handling device is configured to let the gripper grip the lid, lower the second platform and let the gripper move the lid in the horizontal direction over towards the first platform and hold it there during the exposure time, and wherein the plate handling device is configured to let the gripper move the lid in the horizontal direction back to the second platform, raise the second platform, and to release the lid onto the plate to end exposure of the nutrient medium of the plate.

Furthermore, according to an embodiment of the plate handling device, for exposing the nutrient medium of a plate arranged on the second platform, the plate handling device is configured to raise the second platform with the plate positioned thereon and without its lid to an exposure position, and to lower the second platform after an end of the exposure time.

According to yet another embodiment of the plate handling device, the plate handling device is configured to let the gripper move (particularly in the horizontal direction) each plate of a stack of plates carried by the first platform to the second platform, particularly one after the other starting with the topmost plate, to arrange it on the second platform or on a previous plate already arranged on the second platform, to remove the lid of the current plate moved to the second platform with help of the gripper to expose its nutrient medium to the ambient atmosphere for a pre-defined exposure time, to move the lid back towards the first platform with the gripper to hold it there during the exposure time, and to let the gripper move the lid in the horizontal direction back to the second platform to release the lid onto the current plate until all the plates have been moved from the first platform to the second platform and their media have been exposed. I.e., eventually all plates form a stack on the second platform.

Further, in an embodiment of the plate handling device, the plate handling device comprises an electronic control unit, wherein particularly the electronic control unit is configured to control a vertical movement of the first platform and the second platform, a horizontal movement of the gripper, and a gripping movement of the gripper.

According to a further embodiment of the plate handling device, the plate handling device comprises a first optical sensor arranged such that a plate arranged on the first platform can be moved past the sensor with the first platform in the vertical direction, wherein particularly the electronic control unit is configured to receive an output signal of the first optical sensor and to determine based on the output signal of the first optical sensor at least one of: a vertical position of the topmost plate, a number of plates loaded onto the first platform, a loss of a plate and/or lid, a movement of a plate with or without lid.

Furthermore, according to an embodiment of the plate handling device, the plate handling device comprises a second optical sensor arranged such that a plate arranged on the second platform can be moved past the sensor with the second platform in the vertical direction, wherein particularly the electronic control unit is configured to receive an output signal of the second optical sensor and to determine based on the output signal of the second optical sensor at least one of: a number of plates loaded onto the second platform, a loss of a plate and/or lid, a movement of a plate with or without lid.

Further, according to an embodiment of the plate handling device, the plate handling device comprises a memory, and wherein the electronic control unit is configured to store an internal device log indicative of operations performed by the first platform, second platform, and the gripper in the memory, particularly in real-time.

According to yet another embodiment of the plate handling device, the gripper comprises a first and a second gripper arm, the gripper arms being configured to be moved towards one another to grip a plate and/or lid, and to move away from one another to release a gripped plate and/or lid. Particularly, in an embodiment, the respective gripper arm comprises a curved surface comprising at least two protrusions for gripping a plate and/or lid. According to a further embodiment, the respective gripper arm is elastic to accommodate for a range of different outer diameters of a plate and/or lid. Furthermore, according to yet a further embodiment of the plate handling device, the gripper is configured to maintain a relative position between the gripper arms (i.e. a constant distance between the gripper arms) to keep holding a lid or a plate. Particularly, the gripper is configured to move both gripper arms while maintaining said relative position for moving a lid or plate held by the gripper, particularly gripper arms.

Furthermore, according to an embodiment of the plate handling device, the plate handling device comprises a first actuator, particularly a spindle drive, to move the first platform in the vertical direction, and/or wherein the plate handling device comprises a second actuator, particularly a spindle drive, to move the second platform in the vertical direction. Particularly, in the context of the present invention, a spindle drive comprises an actuator such as an electric motor that drives a threaded spindle. Depending on the direction of rotation of the actuator, the spindle turns clockwise or anticlockwise.

According to a further embodiment of the plate handling device, the plate handling device comprises a first gripper actuator, particularly in the form of a spindle drive, configured to move the first gripper arm in the horizontal direction, and wherein the plate handling device comprises a second gripper actuator, particularly in form of a spindle drive, configured to move the second gripper arm in the horizontal direction thereby allowing to move the gripper arms together in the horizontal direction being spaced apart by a constant distance, or towards or away from one another in the horizontal direction thereby facilitating gripping or release of a plate and/or lid. Particularly, the actuators used for moving the platforms and the gripper arms can be identical making production of the plate handling device more efficient.

Further, in an embodiment, the plate handling device comprises a first guiding structure configured to center at least one plate when the at least one plate is arranged on the first platform. Similarly, in a further embodiment, the plate handling device comprises a second guiding structure configured to center at least one plate when the at least one plate is arranged on the second platform. Such guiding structures are particularly helpful in case the respective first or second platform moves a stack of plates up or down as the plates in the stack become centered in a horizontal plane with respect to vertical axis along which they are stacked when the respective platform is moved and individual plates contact the associated guiding structure. The respective guiding structure can comprise rails extending in the vertical direction, respectively.

According to yet another embodiment of the plate handling device, the latter comprises a housing, wherein the first platform and the second platform are moveable up and down in a vertical direction along a front side of the housing. Particularly the housing accommodates the afore-described actuators by means of which the first and second platform as well as the gripper are moved. Furthermore, the first and second guiding structures can be connected to the housing, particularly to the front side of the housing.

According to a further embodiment, the housing comprises at least one foot for supporting the housing on a surface, wherein one or several electrical leads for supplying the plate handling device with electrical energy and/or a control signal and/or for providing communication between the plate handling device and a further device is/are accommodated by the foot via which the respective electrical lead is introduced into an interior space of the housing.

Furthermore, according to an embodiment of the plate handling device, the plate handling device comprises an electronic control unit and a memory operatively connected thereto, wherein the memory is adapted to store a log data indicative of actions performed by the plate handling device. Furthermore, in an embodiment, the plate handling device comprises a communication interface configured to allow integration into and control by a superordinate system. Particularly, the electronic control unit is configured to prompt the plate handling device to expose subsequent plates at the exact same spatial position described by X, Y, and Z coordinates.

Particularly, the plate handling device according to the present invention can achieve a lower contamination risk as only a minimal surface (e.g. the respective plate and the gripper, particularly its two gripper arms) are moving perpendicular to the air flow, reducing disturbance of the laminar air flow and therefore reducing the risk of causing turbulences of the air flow. Furthermore, in an embodiment, the handling of plates does not expose potentially non-decontaminated spaces into the decontaminated environment. Particularly, the plate handler according to the present invention can be configured to be subject to decontamination with loaded plates. Furthermore, in an embodiment, the plate handler according to the present invention is configured to mimic a manual plate handling process and does therefore not flip-over any opened agar plate, i.e., one plate is exposed at a time at the same position.

Furthermore, storing plates vertically on top of each other on two separate positions allows for handling of multiple plates on a minimal footprint. It is therefore feasible to cover multiple passive environmental monitoring positions with multiple instruments, i.e., plate handling devices, of small footprint.

Further, vertical stages as particularly used by the present invention, allow realization of an exposure position located up and above the actual body or housing of the plate handler device - this is in line with a concept called "first air contact".

Furthermore, particularly, the plate movement generated by the plate handling device according to the present invention causes minimal influence on the air flow pattern of the environment (e.g. isolator) in which the plate handling device is used compared to moving human or robotic arms or rotating instrument covers.

Furthermore, using the plate handling device according to the present invention, the standard process of decontaminating the environment and the containment of fresh nutrient plates (typically a plastic envelope) before using these for sampling purposes can be maintained. Particularly, the plate handling device can be completely decontaminated prior to the critical process.

According to a further aspect of the present invention, a microbial air sampling device is disclosed. Particularly, while the plate handling device can be used to sample microbes on the respective exposed plate by exposing the respective plate to an atmosphere surrounding the plate, e.g. in some sort of a room or chamber in which the plate handling device resides, the plate handling device can however also form an integral part of device that actively samples air in an environment.

The microbial air sampling device according to the invention thus comprises a plate handling device according to the present invention, wherein the microbial air sampling device is configured to let an air flow pass along the plate whose lid that has been removed by the plate handling device for collecting microbes comprised by the air flow on the plate.

Particularly, according to an embodiment, the microbial air sampling device comprises a housing comprising a chamber for receiving the plate (particularly when the plate is in said exposure position), the chamber being configured to guide the air flow along the plate.

Furthermore, in an embodiment, the microbial air sampling device comprises an air flow generating device (e.g. a fan) configured to generate the air flow to be passed along the plate.

Furthermore, in an embodiment of the microbial air flow device, the chamber forms part of an air flow channel having an inlet for introducing the air flow into the chamber and an outlet for discharging the air flow out of the microbial air sampling device. Furthermore, in an embodiment, the microbial air sampling device comprise a control unit for controlling the air flow generating device so as to pass a desired volume flow or a desired mass flow of air along the plate per unit time.

According to a further aspect of the present invention, a method for handling plates (particularly using a plate handling device according to the present invention) is disclosed, each plate accommodating a nutrient medium and being covered by a lid, wherein the method comprises moving at least one plate from the first platform to the second platform with the gripper and removing the lid of the at least one plate (particularly with help of the gripper) prior to or after moving the plate from the first platform to the second platform to expose its nutrient medium to a surrounding atmosphere for a pre-defined exposure time.

According to an embodiment of the method, the method can comprise one, several or all of the following steps:
a) arranging a stack of plates on the first platform, each plate accommodating a nutrient medium and being covered by a lid,
b) gripping the topmost plate of the stack of plates with the gripper,
c) moving the plate with the gripper towards the second platform,
d) releasing the plate from the gripper onto the second platform,
e) gripping the lid of the plate with the gripper,
f) moving the lid with the gripper towards the first platform to expose the nutrient medium of the plate for a pre-defined exposure time and holding the lid with the gripper for the entire exposure time,
g) raising the second platform for moving the plate without the lid upwards to expose the nutrient media to an ambient atmosphere,
h) lowering the second platform to move the plate down after the exposure time has lapsed,
i) moving the gripper towards the second platform and raising the second platform to move the plate up underneath the lid,
j) releasing the lid from the gripper onto the exposed plate, and particularly lowering the second platform to move the plate with lid,
k) moving the gripper towards the first platform,
l) continuing with step b) until nutrient media of all plates have been exposed and all plates are arranged in form of a stack on the second platform.

Particularly, prior to step a) the first platform can be moved into a first position (e.g. upwards for easy positioning of the plates on the first platform).

Furthermore, once the handling process is finished and all plates are processed, the platform holding the plates can be moved to an unloading position (e.g. upwards for easy plate retrieval).

According to a further embodiment of the method step a) further comprises moving the stack of plates down with the first platform past a first optical sensor of the plate handling device to detect the stack of plates and calculate the number of plates in the stack of plates, and raising the first platform such that the topmost plate can be gripped by the gripper.

Furthermore, according to an embodiment of the method, step b) further comprises lowering the first platform past the first optical sensor to determine a height of the stack of plates and verify that the topmost plate was successfully gripped.

Further, according to yet a further embodiment of the method, step c) further comprises lowering the second platform past a second optical sensor of the plate handling device to determine a height of a stack of plates arranged on the second platform, and raising the second platform underneath the plate.

In a further embodiment of the method, step d) further comprises lowering the second platform past the second optical sensor to determine a height of a stack of plates arranged on the second platform and to confirm that the plate has been moved from the first platform to the second platform.

Furthermore, according to an embodiment of the method, step e) further comprises raising the second platform to move the plate up and gripping its lid with the gripper, and moving the plate down with the second platform past the second optical sensor to determine a height of a stack of plates arranged on the second platform and to confirm that the lid was removed.

Furthermore, according to an embodiment of the method, step h) further comprises, after exposure, moving the plate down past the second optical sensor with the second platform to detect if the plate is still present.

Furthermore, according to an embodiment of the method, step j) further comprises moving the second platform down past the second optical sensor to detect that the lid was correctly placed on the plate.

Furthermore, according to an embodiment of the method, step k) further comprises moving the first platform down past the first optical sensor to detect a height of a stack of plates arranged on the first platform to determine if the number of plates has decreased by one (since the last exposure of the previous plate).

Regarding steps a) to I) of the above described embodiment of the present invention, the respective plate can be moved from the first platform to the second platform wherein the lid of the plate can be removed when the plate is on the second platform. Particularly, this means that exposure of the plate to the adjacent atmosphere is conducted on the second platform. However, according to a further embodiment, the lid of the respective (e.g. topmost) plate can be removed when the plate is residing on the second platform and exposure of the plate to the atmosphere is then conducted on the first platform. The lid can than be placed on the plate with the plate still residing on the first platform. Afterwards the plate can be moved to the second platform with its lid placed on the plate. In this way, the plate handling device can cycle through a number of plates as well. Particularly, these two ways of exposing the respective plate can be applied to all embodiments of the method and plate handling device according to the present invention described herein.

Furthermore, the individual embodiments and features described herein in conjunction with the plate handling device according to the present invention may be used to further specify the method according to the present invention and vice versa.

In the following, embodiments of the present invention as well further features and advantages and other aspects of the present invention shall be described with reference to the Figures, wherein
- Fig. 1: shows a schematic view onto a front side of an embodiment of a plate handling device according to the present invention,
- Fig. 2A: shows a top view onto an embodiment of a gripper of the plate handling device comprising two gripper arms that can be moved towards and away from one another as well as together to move the whole gripper,
- Fig. 2B: shows the gripper as shown in Fig. 2A in the process of gripping a plate,
- Fig. 3A to 3F: shows an illustration of an embodiment of a method according to the present invention, and
- Fig. 4: shows an embodiment of a further aspect of the present application relating to an active microbial air sampling device utilizing a plate handling device according to the present invention.

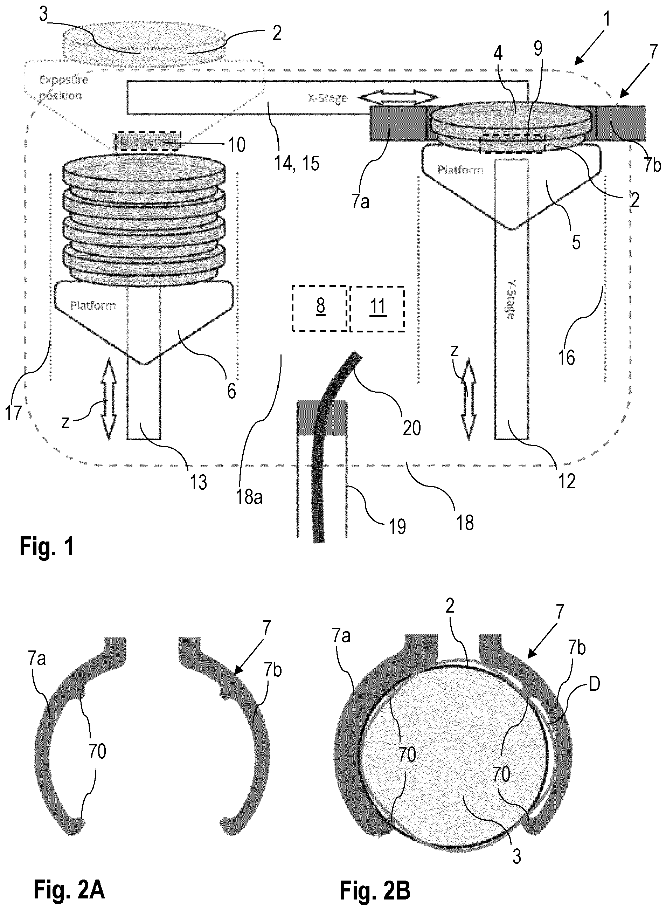

Fig. 1 shows in conjunction with Figs. 2A and B an embodiment of a plate handling device according to the present invention. The plate handling device 1 is configured to automatically handle plates 2 accommodating a nutrient medium 3 and being covered by a lid 4. Such plates 2 are used to facilitate continuous microbial contamination monitoring during critical processes. Particularly, following gravitational forces particles/microbes can settle onto the exposed nutrient medium carried by the plate 2. Whenever processes exceed the maximum test time for a single plate 2, exchange of the nutrient media plate 2 is required. One key reason for the plate exchange is the natural evaporation of the water from the medium when exposed to the ambient environment. The environment to be tested may be a pharmaceutical production line for drugs, or in medical device fabrication, foodstuff production facilities, restaurants, industries, healthcare facilities, etc. and typical exposure time limits for a single plate 2 are around 240min. As the plate exchanges are mostly performed manually, this often requires pausing the production process due to manual intervention. The present invention avoids this disadvantage by being able to automatically handling a plurality of plates 2 that can be exposed one after the other. Particularly, to this end, the plate handling device 1 comprises a first platform being 5 moveable up and down in a vertical direction z and configured to carry a plurality of said plates 2 stacked on top of one another, a second platform 6 being moveable up and down in a vertical direction z laterally next to the first platform 5, and a gripper 7 being moveable back and forth in a horizontal direction z to operatively interact with plates 2 on the first and the second platform 5, 6. Furthermore, the plate handling device 1 is particularly configured to let the gripper 7 grip a (e.g. topmost) plate 2 carried by the first platform 5, move this plate 2 (e.g. in the horizontal direction x) to the second platform 6 and to arrange it thereon. Further, the plate handling device 1 is further configured to remove the lid 4 of this plate 2, e.g., with help of the gripper 7, to expose its nutrient medium 3 to the ambient atmosphere for a pre-defined exposure time. For removing the lid 4, the plate handling device 1 can be configured to let the gripper 7 grip the lid 4, lower the second platform 6 and let the gripper 7 move the lid 4 in the horizontal direction x over towards the first platform 5 and hold it there during the exposure time as shown in Fig. 1. The lid 4 may also be temporarily stored in other ways. Furthermore, as already described above, it is also conceivable in an alternative embodiment to expose the respective plate 2 already on the first platform, close it with its lid 4 afterwards and transfer the closed plate 2 (e.g. with lid 4 thereon) to the second platform for storage.

Furthermore, as also indicated in Fig. 1, for exposing the nutrient medium 3 of the opened plate 2 arranged on the second platform 6, the plate handling device 1 is configured to raise the second platform 6 with the plate 2 positioned thereon and without its lid 4 to an exposure position. The plate handling device 1 is further adapted to lower the second platform 6 with the plate 2 thereon after the exposure time has ended and to let the gripper 7 move the lid 4 in the horizontal direction x back to the second platform 6, raise the second platform 6, and to release the lid 4 onto the plate 2 to end exposure of the nutrient medium 3 of the plate 2. In the same fashion, the plate handling device can cycle through the other plates 2 stacked on the first platform 5 until all plates have been exposed and are stacked with their lids 4 on the second platform 6.

For controlling the above-described operations of the plate handling device 1, the latter comprises an electronic control unit 8 and can further comprise a memory 11 configured to store an internal device log indicative of operations performed by the plate handling device.

Furthermore, the plate handling device 1 can comprise a first optical sensor 9 arranged such that a plate 2 arranged on the first platform 5 can be moved past the first optical sensor 9 by means of the first platform 5 in the vertical direction z, wherein particularly the electronic control unit 8 is configured to receive an output signal of the first optical sensor 9 and to determine based on the output signal of the first optical sensor 9 at least one of: a number of plates loaded onto the first platform 5, a loss of a plate 2 and/or lid 4, a movement of a plate 2 with or without lid 4. In the same manner, the plate handling device 1 can comprise a second optical sensor 10 arranged such that a plate 2 arranged on the second platform 6 can be moved past the second optical sensor 10 by means of the second platform 6 in the vertical direction z, wherein particularly the electronic control unit 8 is configured to receive an output signal of the second optical sensor 10 and to determine based on the output signal of the second optical sensor 10 at least one of: a number of plates 2 loaded onto the second platform, a loss of a plate 2 and/or lid 4, a movement of a plate 2 with or without lid 4.

Further, in an embodiment of the invention shown in Figs. 2A and 2B, the gripper 7 comprises a first and a second gripper arm 7a, 7b, the gripper arms 7a, 7b being configured to be moved towards one another to grip a plate 2 and/or lid 4, and to move away from one another to release a gripped plate 2 and/or lid 4. Particularly, each gripper arm 7a, 7b can be moved horizontally along direction 6 with a dedicated actuator 14, 15, wherein the respective actuator can be a spindle drive. Particularly, all actuators 12-15 of the device 1 can be identical making production of the device 1 more efficient. Furthermore, the arms 7a, 7b can be moved while maintaining a constant distance to one another resulting in a displacement of the whole gripper 7 along the horizontal direction 6. Furthermore, as indicated particularly in Fig. 2B, the respective gripper arm 7a, 7b can comprises a curved surface that can comprise at least two protrusions 70 for gripping a plate 2 and/or lid 4, wherein particularly the respective gripper arm 7a, 7b is elastic to accommodate for a range of different outer diameters D of a plate 2 and/or lid 4 as schematically indicated by the deformed circular line in Fig. 2B.

The design according to the invention involving in particular two vertical plate stacks that are moved using two vertical stages 12, 13 and one gripper 7 comprising two independent arms each on one horizontal stage 14, 15 allows an efficient automatic plate handling. As described above, the gripper 7 realizes the gipping functions for the respective plate 2 as well as the corresponding lid 4 and horizontal movement of plates 2 from one side of the device 1 to the other side. Furthermore, on the bottom of the plate handling device 1 a cable feedthrough 19 is provided that can be formed by a foot of a housing 18 of the device 1. This setup can be used to combine an (e.g. hermetic) cable feed-through with the mechanical stand and thereby reduces potential impact of the surrounding isolator environment in which the device 1 may be used.

As the two horizontal axes/stages 14, 15 and arms 7a, 7b combined form the gripper 7 that can be maneuvered horizontally, there is no need to place one actuator unit atop of a moving linear stage thus removing the need to provide and protect a moving electrical connection. Both vertical stages 12, 13 are equipped with said optical sensors 9, 10 to detect the presence of a plate 2. In combination with the position and movement of the respective platform 5, 6, the number of plates 2 and their height on either of the two platforms 5, 6 can be determined. Particularly, all electrical connections are routed from the monitored environment (pharmaceutical isolator unit) through one foot/mounting 19 which can be hermetically sealed while the system is otherwise designed to allow air flow passing through the device 1 for improved gas decontamination.

Furthermore, in an embodiment, the fully automated as well as manual control options using either a remote interface command set or an integrated button enable versatile use cases in partially or fully automated settings.

Further, in an embodiment, the internal electronic control unit 8 stores all handling operations and sampling processes in a device log together with their timestamp in a memory 11 in order to allow traceability of plates and improve data integrity. Optical and electronic indications can furthermore signal the device status to the used or superordinate control system to enable a quick response.

Furthermore, according to an embodiment, the plate handling device 1 is adapted to undergo volatile hydrogen peroxide (VHP) decontamination as its operating environment is usually disinfected and decontaminated regularly using gaseous or liquid chemical agents. This requires all sensor and actuator units as well as electronics of the device 1 to be compatible with such environments. Especially, said VHP decontamination has gained popularity in the last years. Similarly, the gaseous decontamination agent must be able to reach all locations, voids or internal chambers of parts or instruments in the isolator unit to guarantee high confidence in the success of the decontamination process. Especially, sealed compartments that are non-exposed to the decontamination agent and opened during the use of the isolator pose severe risks on the effectiveness of the decontamination process and have to be avoided.

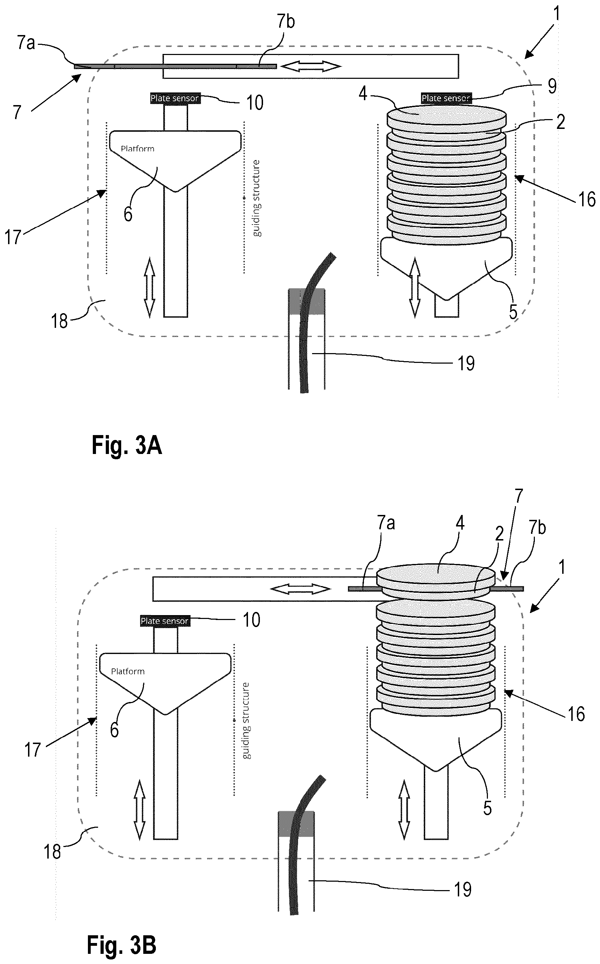

Further, Fig. 3A to Fig. 3F illustrate an embodiment of a method for automatically handling plates, particularly using a plate handling device 1 according to the present invention.

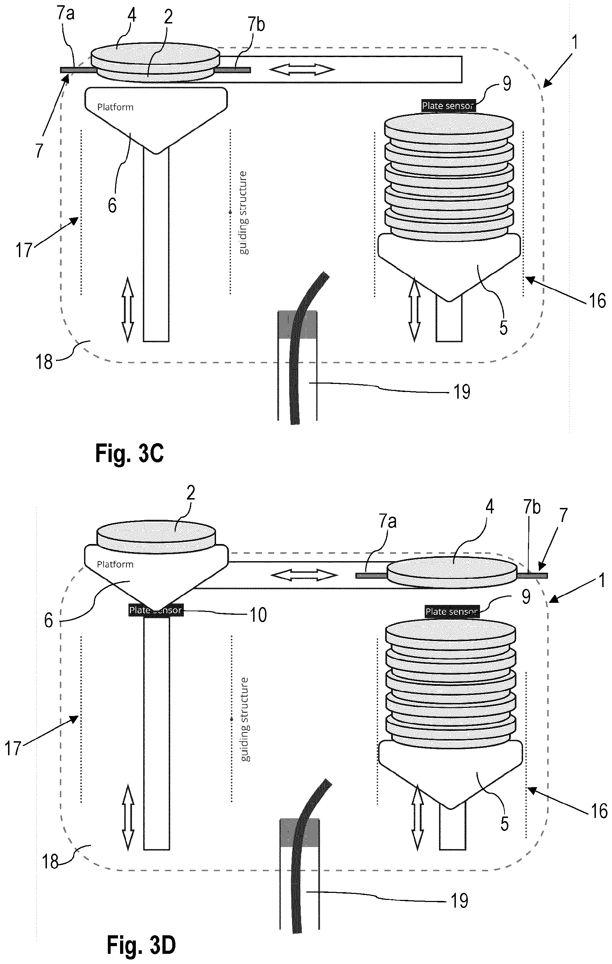

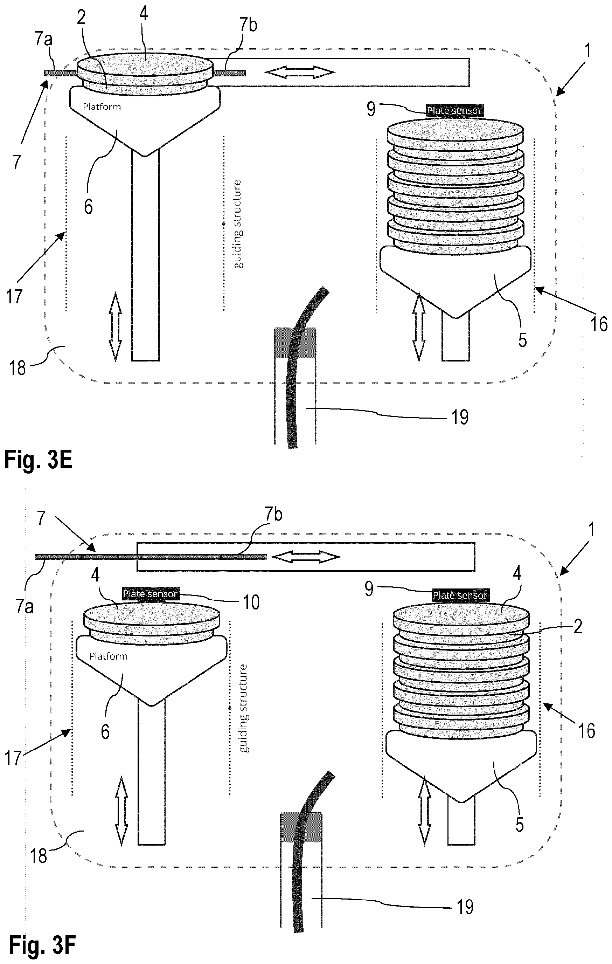

As indicated in Fig. 3A, a stack of nutrient plates 2 is loaded to the first platform 5 of the device 1. A first guiding structure 16 may facilitate alignment of multiple plates 2 on top of each other. As shown in Fig. 3B, the stack of plates 2 was moved upwards using the first optical sensor 9 and the gripper 7 is closed around the nutrient plate 2. Fig. 3C shows the plate's 2 position after movement of the gripper 7 to the left side of the device 1. There, the plate 2 is subsequently released onto the second platform 6. Fig. 3D shows the plate 2 was lowered on second platform 6 to first grip the plate's lid 4 and then lower the plate 2 even further to allow movement of the sole lid 4 to the right side of the device 1 and an upwards movement of the plate 2 on the second platform 6 into the exposure position. Now particles/microbes can settle on the nutrient medium contained by the plate 2. Fig. 3E further indicates that the plate 2 has been taken down from the exposure position and the lid 4 was placed back onto it. At this moment the handling of the first plate 2 is finished. Fig. 3F displays the status of the device 1 after movement of the gripper 7 to the first platform 5 and lowering of the exposed plate 2 as prerequisite for the next handling iteration of the now topmost nutrient plate 2 residing on the first platform 5.

Particularly, the individual steps that the plate handling device 1 can conduct can be summarized as follows:
- Moving the device 1 to its load position (e.g. the first platform 5 can be moved upwards for easy loading of the plates 2),
- Loading plates 2 on the first platform 5,
- Triggering the start of the process (e.g. locally or remotely),
- Moving the stack of plates 2 down past the first optical sensor 9 and particularly determining the plate stack and calculating the number of loaded plates 2,
- Moving the first platform 5 up such that the topmost plate 2 is arranged in between the gripper arms 7a, 7b,
- Closing the gripper 7 to grip the first/topmost plate 2,
- Moving the first platform 5 down past the first optical sensor 9 and determining the plate stack height and verify that one plate 2 was successfully gripped (e.g. not just a lid 4),
- Moving the plate 2 to the second platform 6,
- Moving the second platform 6 down past the second optical sensor 10 to detect the stack height and moving the second platform 6 up very slightly underneath the plate 2,
- Opening the gripper 7 to release the plate 2 onto the second platform 6,
- Moving the second platform 6 down past the first optical sensor 10 and determining the plate stack height and confirming that the plate 2 has been moved from first platform 5 to the second platform 6,
- Moving the plate 2 upwards by means of the second platform 6 and closing the gripper 7 to grip the lid 4,
- Moving the plate 2 down past the second optical sensor 10 and determining the plate stack height and confirming that the lid 4 was removed,
- Moving the lid 4 to the first platform 5 and holding it with the gripper 7 for the entire exposure time,
- Moving the plate 2 without lid 4 by means of the second platform 6 upwards to be exposed on a highest point of the second platform 6 which corresponds to the first air contact of the exposed plate 2,
- After exposing the plate 2, moving the plate 2 down with the second platform 6 past the second optical sensor 10 and detecting if the plate 2 is still present,
- Moving the gripper 7 to the side of the second platform 6 and moving the second platform 6 up very slightly underneath the lid 4,
- Opening the gripper 7 to release the lid 4 onto the exposed plate 2,
- Moving the second platform 6 down past the second optical sensor 10 and detecting that the lid 4 was correctly placed on the plate 2,
- Moving the gripper 7 to the side of the first platform 5,
- Moving the first platform 5 down past the first optical sensor 9 and detecting the plate stack height and inferring if it does match with the last exposure round minus one plate,
- Continuing process with the next plate 2 from the loaded stack on the first platform 5.

Once the handling process is finished and all plates 2 are processed, the device 1 can be moved to an unloading position (e.g. the second platform 6 can be moved upwards for easy plate retrieval). As described before, the above-described steps can be modified by removing the lid 4 of the respective (e.g. topmost) plate 2 already on the first platform 5, exposing the respective plate 2 on the second platform 6 and transferring the plate 2 with lid thereon to the second platform 6, wherein also here the gripper can be used for removing the lid 4 on the plate 2, placing it on the plate 2, and for moving the respective plate 2 from the first to the second platform 5, 6.

The present invention offers several advantages over the prior art. Particularly, using multiple linear axes in one enclosure to move and grip nutrient plates 2 as well as their lids 4 for automated passive environmental monitoring proves to beneficial as no single axis needs to be stacked onto another moving part reducing the risk of cable damage and reducing the overall system complexity.

Further, the use of positional and optical sensors to determine and track plate positions during the handling routine enables quick failure recognition and error reporting in case of faults.

Furthermore, particularly, the plate handling device can use its optical sensors to detect the number of plates 2 loaded to each platform 5, 6. Also determining the plate count after each handling step allows the detection of plate or lid loss for improved reliability during the handling workflow and quick error detection.

Further, using the aforementioned optical sensors, the plate stack on both vertically moving platforms 5, 6 can be moved past their individual optical sensors to determine the stack height on both platforms 5, 6 of the device 1. Using this information, the electronic control unit/internal control software can recognize if a plate with lid or without lid was moved or detect situations where a lid or plate were lost. Using a force feedback for these use cases would require constant load on the lid/plate that is gripped and therefore requires constant power consumption even during the long exposure durations.

Furthermore, an improved traceability and data integrity of the passive environmental monitoring processes is achieved as all handling operations can be stored in an internal device log. This is achieved by using an internal control logic and memory that stores all handling steps as they are happening into a dedicated technical log together with the time stamp of the particular step.

An integrated specialized solution for plate handling instead of using a multi-purpose robotic arm or even manual workflows enables the optimization of the gripper geometry for more reliable and robust plate and lid handling. It also enables the optimization of the gripper design towards compatibility to a wide range of nutrient plate diameters and shapes.

Furthermore, the gripper geometry enables high tolerance to imprecisions of the actual horizontal axis movements. Therefore, the design can take advantage of the elasticity of the polymer materials used in classical single use nutrient plates. Using protruding contact points, plate deformation can be leveraged to compensate for slight deviations in the plate diameter or movement errors of few millimeters which is more than sufficient for a broad range of 90mm media plates.

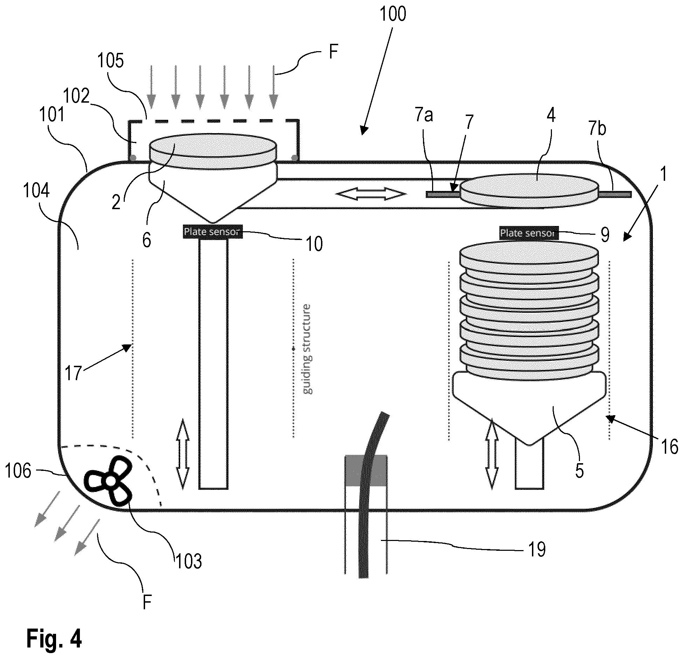

Fig. 4 shows a further application of a plate handling device according to the present invention. Particularly, the plate handling device according to the present invention can be used as a stand-alone-device, e.g. in order to measure air quality in a confined room or space (e.g. of laboratory equipment) in which the respective plate 2 is exposed to the atmosphere by the plate handling device. However, a plate handling device or a corresponding assembly may also form an integral part of a device that actively samples air in an environment such as a microbial air sampler 100 depicted in Fig. 4.

According thereto, this embodiment of a microbial air sampling device 100 comprises a plate handling device 1 according to the present invention, wherein the microbial air sampling device 100 is configured to let an air flow F pass along the plate 2 whose lid 4 has been removed by the plate handling device 1 for collecting microbes comprised by the air flow F on the plate 2 (the plate 2 can comprise a nutrient medium, e.g. the plate can be an agar plate).

The microbial air sampling device 100 can comprise a housing 101 defining a chamber 102 for receiving the plate 2 that is to be exposed to the air flow F, wherein the chamber 102 is configured to guide the air flow F along the plate 2. Particularly, the chamber 102 can be located at the exposure position to which the plate handling device 1 can move the plate 2 to expose the latter to the air flow (with the lid 4 being removed in before).

Particularly, for actively sampling the air in an environment in which the microbial air sampling device 100 is located, the microbial air sampling device 100 can comprise an air flow generating device (e.g. a fan) 103 configured to generate the air flow F to be passed along the plate 2 residing in chamber 102.

Furthermore, the chamber 102 can form part of an air flow channel 104 having an inlet 105 for introducing the air flow F into the chamber 102 and an outlet 106 for discharging the air flow F out of the microbial air sampling device 100. Further, the microbial air sampling device 100 can comprise a control unit for controlling the air flow generating device 103 so as to pass a desired volume flow or a desired mass flow of air along the plate 2 per unit time.

As before, the microbial air sampling device 100 can cycle through a plurality of plates 2 by e.g. moving the respective plate 2 with removed lid 4 into the chamber 102 and exchanging it with a new plate 2 by means of the integrated plate handling device 1 after its exposure.

## Claims

1. A plate handling device (1) for handling plates (2), the respective plate (2) accommodating a nutrient medium (3) and being covered by a lid (4), wherein the plate handling device (1) comprises:
- a first platform (5) being moveable up and down in a vertical direction (z) and configured to carry at least one plate (2) or a plurality of plates (2) stacked on top of one another,
- a second platform (6) being moveable up and down in a vertical direction (z), and
- a movable gripper (7), wherein the plate handling device (1) is configured to let the gripper (7) grip a plate (2) carried by the first platform (5), move the plate (2) to the second platform (6) and to arrange it thereon, and wherein the plate handling device (1) is further configured to remove the lid (4) of the plate (2) for exposing its nutrient medium (3) to an atmosphere for a pre-defined exposure time.

2. The plate handling device according to claim 1, wherein for removing the lid (4), the plate handling device (1) is configured to let the gripper (7) grip the lid (4), move the lid (4) away from the plate (2) and hold it during the exposure time or arrange it on a another location to remain there during the exposure time, and wherein the plate handling device (1) is configured to let the gripper (7) move the lid (4) back to the plate (2) and to release the lid (4) onto the plate (2).

3. The plate handling device according to claim 1 or 2, wherein for exposing the nutrient medium (3) of a plate (2) arranged on the first or second platform (5, 6), the plate handling device (1) is configured to raise the first or second platform (5, 6) with the plate (2) positioned thereon and without its lid (4) to an exposure position, and to lower the first or second platform (5, 6) after an end of the exposure time.

4. The plate handling device according to one of the preceding claims, wherein the plate handling device (1) is configured to let the gripper (7) move each plate (2) of a stack of plates (2) carried by the first platform (5) to the second platform (6), to arrange it thereon or on a previous plate already arranged on the second platform (6), to remove the lid (4) of a plate (2) with help of the gripper (7) prior to or after moving the plate (2) from the first platform (5) to the second platform (6) to expose its nutrient medium (3) to the surrounding atmosphere for a pre-defined exposure time, to move the lid (4) away from the plate (2) and hold it during the exposure time or arranged it on another location to remain there during the exposure time, and to let the gripper (7) move the lid (4) back to the plate (2) and to release the lid (4) onto the plate (2).

5. The plate handling device (1) according to one of the preceding claims, wherein the plate handling device (1) comprises an electronic control unit (8), wherein particularly the electronic control (8) unit is configured to control a vertical movement of the first platform (5) and the second platform (6), a horizontal movement of the gripper (7), and a gripping movement of the gripper (7).

6. The plate handling device according to one of the preceding claims, wherein the plate handling device (1) comprises a first optical sensor (9) arranged such that a plate (2) arranged on the first platform (5) can be moved past the first optical sensor (9) with the first platform (5), wherein particularly the electronic control unit (8) is configured to receive an output signal of the first optical sensor (9) and to determine based on the output signal of the first optical sensor (9) at least one of: a number of plates loaded onto the first platform (5), a loss of a plate (2) and/or lid (4), a movement of a plate (2) with or without lid (4).

7. The plate handling device according to one of the preceding claims, wherein the plate handling device (1) comprises a second optical sensor (10) arranged such that a plate (2) arranged on the second platform (6) can be moved past the second optical sensor (10) with the second platform (6), wherein particularly the electronic control unit (8) is configured to receive an output signal of the second optical sensor (10) and to determine based on the output signal of the second optical sensor (10) at least one of: a number of plates loaded onto the second platform (6), a loss of a plate (2) and/or lid (4), a movement of a plate (2) with or without lid (4).

8. The plate handling device according to one of the preceding claims, wherein the plate handling device (1) comprises a memory (11), and wherein the electronic control unit (8) is configured to store an internal device log indicative of operations performed by the plate handling device (1) in the memory (11).

9. The plate handling device according to one of the preceding claims, wherein the gripper (7) comprises a first and a second gripper arm (7a, 7b), the gripper arms (7a, 7b) being configured to be moved towards one another to grip a plate (2) and/or lid (4), and to move away from one another to release a gripped plate (2) and/or lid (4).

10. The plate handling device according to one of the preceding claims, wherein the plate handling device (1) comprises a first actuator (12), particularly a spindle drive, to move the first platform (1) in the vertical direction (z), and/or wherein the plate handling device (1) comprises a second actuator (13), particularly a spindle drive, to move the second platform (6) in the vertical direction (z).

11. The plate handling device according to one of the preceding claims, wherein the plate handling device (1) comprises a first gripper actuator (14), particularly a spindle drive, configured to move the first gripper arm (7a) in the horizontal direction (x), and wherein the plate handling device (1) comprises a second gripper actuator (15), particularly a spindle drive, configured to move the second gripper arm (7b) in the horizontal direction.

12. The plate handling device according to one of the preceding claims, wherein the plate handling device (1) comprises a first guiding structure (16) configured to center at least one plate (2) when the at least one plate (2) is placed on the first platform (5), and/or wherein the plate handling device (1) comprises a second guiding structure (17) configured to center at least one plate (2) when the at least one plate (2) is placed on the second platform (6).

13. A microbial air sampling device (100), comprising a plate handling device (1) according to one of the preceding claims, wherein the microbial air sampling device (100) is configured to let an air flow (F) pass along the plate (2) whose lid (4) has been removed by the plate handling device (1) for collecting microbes comprised by the air flow (F) on said plate (2).

14. A method for handling plates (2) using a plate handling device (1) according to one of the claims 1 to 12, each plate (2) accommodating a nutrient medium (3) and being covered by a lid (4), wherein the method comprises moving at least one plate (2) from the first platform (5) to the second platform (6) with the gripper (7) and removing the lid (4) of the at least one plate (2) prior to or after moving the at least one plate (2) from the first platform (5) to the second platform (6) to expose its nutrient medium (3) to the surrounding atmosphere for a pre-defined exposure time.

15. The method according to claim 14, wherein the method comprises the steps of:
a) arranging a stack of plates (2) on the first platform (5), each plate (2) accommodating a nutrient medium (3) and being covered by a lid (4),
b) gripping the topmost plate (2) of the stack of plates (2) with the gripper (7),
c) moving the plate (2) with the gripper (7) towards the second platform (6),
d) releasing the plate (2) from the gripper (7) onto the second platform (6),
e) gripping the lid (4) of the plate (2) with the gripper (7),
f) moving the lid (4) with the gripper (7) towards the first platform (5) to expose the nutrient medium (3) of the plate (2) for a pre-defined exposure time and holding the lid (4) with the gripper (7) for the entire exposure time,
g) raising the second platform (6) for moving the plate (2) without the lid (4) upwards to expose the nutrient media (3) to an ambient atmosphere,
h) lowering the second platform (6) to move the plate (2) down after the exposure time has lapsed,
i) moving the gripper (7) towards the second platform (6) and raising the second platform (6) to move the plate (2) up underneath the lid (4),
j) releasing the lid (4) from the gripper (7) onto the exposed plate (2), and particularly lowering the second platform (6) with the plate (2) and its lid (4) thereon,
k) moving the gripper towards the first platform (5),
l) continuing with step b), particularly until nutrient media of all plates have been exposed and all plates (2) are arranged in form of a stack on the second platform (6).

## Amended claims

### Amended claims in accordance with Rule 137(2) EPC.

1. A plate handling device (1) for handling plates (2), the respective plate (2) accommodating a nutrient medium (3) and being covered by a lid (4), wherein the plate handling device (1) comprises:
- a first platform (5) being moveable up and down in a vertical direction (z) and configured to carry at least one plate (2) or a plurality of plates (2) stacked on top of one another,
- a second platform (6) being moveable up and down in a vertical direction (z), and
- a movable gripper (7), wherein the plate handling device (1) is configured to let the gripper (7) grip a plate (2) carried by the first platform (5), move the plate (2) to the second platform (6) and to arrange it thereon, and wherein the plate handling device (1) is further configured to remove the lid (4) of the plate (2) for exposing its nutrient medium (3) to an atmosphere for a pre-defined exposure time,
wherein for removing the lid (4), the plate handling device (1) is configured to let the gripper (7) grip the lid (4), move the lid (4) away from the plate (2) and hold it during the exposure time or arrange it on a another location to remain there during the exposure time, and wherein the plate handling device (1) is configured to let the gripper (7) move the lid (4) back to the plate (2) and to release the lid (4) onto the plate (2).

2. The plate handling device according to claim 1, wherein for exposing the nutrient medium (3) of a plate (2) arranged on the first or second platform (5, 6), the plate handling device (1) is configured to raise the first or second platform (5, 6) with the plate (2) positioned thereon and without its lid (4) to an exposure position, and to lower the first or second platform (5, 6) after an end of the exposure time.

3. The plate handling device according to one of the preceding claims, wherein the plate handling device (1) is configured to let the gripper (7) move each plate (2) of a stack of plates (2) carried by the first platform (5) to the second platform (6), to arrange it thereon or on a previous plate already arranged on the second platform (6), to remove the lid (4) of a plate (2) with help of the gripper (7) prior to or after moving the plate (2) from the first platform (5) to the second platform (6) to expose its nutrient medium (3) to the surrounding atmosphere for a pre-defined exposure time, to move the lid (4) away from the plate (2) and hold it during the exposure time or arranged it on another location to remain there during the exposure time, and to let the gripper (7) move the lid (4) back to the plate (2) and to release the lid (4) onto the plate (2).

4. The plate handling device (1) according to one of the preceding claims, wherein the plate handling device (1) comprises an electronic control unit (8), wherein particularly the electronic control (8) unit is configured to control a vertical movement of the first platform (5) and the second platform (6), a horizontal movement of the gripper (7), and a gripping movement of the gripper (7).

5. The plate handling device according to one of the preceding claims, wherein the plate handling device (1) comprises a first optical sensor (9) arranged such that a plate (2) arranged on the first platform (5) can be moved past the first optical sensor (9) with the first platform (5), wherein particularly the electronic control unit (8) is configured to receive an output signal of the first optical sensor (9) and to determine based on the output signal of the first optical sensor (9) at least one of: a number of plates loaded onto the first platform (5), a loss of a plate (2) and/or lid (4), a movement of a plate (2) with or without lid (4).

6. The plate handling device according to one of the preceding claims, wherein the plate handling device (1) comprises a second optical sensor (10) arranged such that a plate (2) arranged on the second platform (6) can be moved past the second optical sensor (10) with the second platform (6), wherein particularly the electronic control unit (8) is configured to receive an output signal of the second optical sensor (10) and to determine based on the output signal of the second optical sensor (10) at least one of: a number of plates loaded onto the second platform (6), a loss of a plate (2) and/or lid (4), a movement of a plate (2) with or without lid (4).

7. The plate handling device according to one of the preceding claims, wherein the plate handling device (1) comprises a memory (11), and wherein the electronic control unit (8) is configured to store an internal device log indicative of operations performed by the plate handling device (1) in the memory (11).

8. The plate handling device according to one of the preceding claims, wherein the gripper (7) comprises a first and a second gripper arm (7a, 7b), the gripper arms (7a, 7b) being configured to be moved towards one another to grip a plate (2) and/or lid (4), and to move away from one another to release a gripped plate (2) and/or lid (4).

9. The plate handling device according to one of the preceding claims, wherein the plate handling device (1) comprises a first actuator (12), particularly a spindle drive, to move the first platform (1) in the vertical direction (z), and/or wherein the plate handling device (1) comprises a second actuator (13), particularly a spindle drive, to move the second platform (6) in the vertical direction (z).

10. The plate handling device according to one of the preceding claims, wherein the plate handling device (1) comprises a first gripper actuator (14), particularly a spindle drive, configured to move the first gripper arm (7a) in the horizontal direction (x), and wherein the plate handling device (1) comprises a second gripper actuator (15), particularly a spindle drive, configured to move the second gripper arm (7b) in the horizontal direction.

11. The plate handling device according to one of the preceding claims, wherein the plate handling device (1) comprises a first guiding structure (16) configured to center at least one plate (2) when the at least one plate (2) is placed on the first platform (5), and/or wherein the plate handling device (1) comprises a second guiding structure (17) configured to center at least one plate (2) when the at least one plate (2) is placed on the second platform (6).

12. A microbial air sampling device (100), comprising a plate handling device (1) according to one of the preceding claims, wherein the microbial air sampling device (100) is configured to let an air flow (F) pass along the plate (2) whose lid (4) has been removed by the plate handling device (1) for collecting microbes comprised by the air flow (F) on said plate (2).

13. A method for handling plates (2) using a plate handling device (1) according to one of the claims 1 to 11, each plate (2) accommodating a nutrient medium (3) and being covered by a lid (4), wherein the method comprises moving at least one plate (2) from the first platform (5) to the second platform (6) with the gripper (7) and removing the lid (4) of the at least one plate (2) prior to or after moving the at least one plate (2) from the first platform (5) to the second platform (6) to expose its nutrient medium (3) to the surrounding atmosphere for a pre-defined exposure time.

14. The method according to claim 13, wherein the method comprises the steps of:
a) arranging a stack of plates (2) on the first platform (5), each plate (2) accommodating a nutrient medium (3) and being covered by a lid (4),
b) gripping the topmost plate (2) of the stack of plates (2) with the gripper (7),
c) moving the plate (2) with the gripper (7) towards the second platform (6),
d) releasing the plate (2) from the gripper (7) onto the second platform (6),
e) gripping the lid (4) of the plate (2) with the gripper (7),
f) moving the lid (4) with the gripper (7) towards the first platform (5) to expose the nutrient medium (3) of the plate (2) for a pre-defined exposure time and holding the lid (4) with the gripper (7) for the entire exposure time,
g) raising the second platform (6) for moving the plate (2) without the lid (4) upwards to expose the nutrient media (3) to an ambient atmosphere,
h) lowering the second platform (6) to move the plate (2) down after the exposure time has lapsed,
i) moving the gripper (7) towards the second platform (6) and raising the second platform (6) to move the plate (2) up underneath the lid (4),
j) releasing the lid (4) from the gripper (7) onto the exposed plate (2), and particularly lowering the second platform (6) with the plate (2) and its lid (4) thereon,
k) moving the gripper towards the first platform (5),
l) continuing with step b), particularly until nutrient media of all plates have been exposed and all plates (2) are arranged in form of a stack on the second platform (6).
